# EUROPEAN PATENT SPECIFICATION

(11) **EP 1 002 794 B1**
(45) Date of publication and mention of the grant of the patent: **04.02.2009**
(21) Application number: 99500208.6
(22) Date of filing: 10.11.1999
(51) Int. Cl.: C07D 211/22, A61K 31/4465

(54) **4-¬(Aryl)(aryloxy)methyl piperidine derivatives and their use as serotonin and/or noradrenaline reuptake inhibitors**
4-¬(Aryl)(aryloxy)methyl piperidinderivate und ihre Verwendung als Serotonin und/oder Noradrenalin Reuptake Inhibitoren
Dérivés de 4-¬(aryl)(aryloxy)methyl pipéridine et leur usage comme inhibiteurs de la réabsorption de sérotonine et/ou de noradrénaline

(30) Priority: 18.11.1998 ES 9802420
(43) Date of publication of application: 24.05.2000
(73) Proprietor: FAES, Fabrica Espanola de Productos Quimicos y Farmaceuticos, S.A., 48940 Lejona, Vizcaya (ES)
(72) Inventor: Orjales Venero, Aurelio, 48990 Neguri (Vizcaya) (ES); Toledo Avello, Antonio, 48990 Algorta (Vizcaya) (ES); Pumar Duran, Carmen, 48110 Gatika (Vizcaya) (ES)
(74) Representative: ABG Patentes, S.L.

(56) References cited:
- MELLONI P ET AL: "Potential antidepressant agents. Alpha-Aryloxy-benzyl derivatives of ethanolamine and morpholine" EUROPEAN JOURNAL OF MEDICINAL CHEMISTRY - CHIMICA THERAPEUTICA, vol. 19, no. 3, 1 January 1984 (1984-01-01), pages 235-242, XP000605241 ISSN: 0223-5234
- DATABASE WPI Section Ch, Week 199616 Derwent Publications Ltd., London, GB; Class B05, AN 1996-157056 XP002130616 -& JP 08 040999 A (YAMANOUCHI PHARM CO LTD), 13 February 1996 (1996-02-13)
- CHEMICAL ABSTRACTS, vol. 106, no. 25, 22 June 1987 (1987-06-22) Columbus, Ohio, US; abstract no. 213767z, SUGIMOTO H ET AL: "Piperidine derivatives" page 645; column 2; XP002130615 -& JP 61 227565 A (EISAI CO LTD) 9 October 1986 (1986-10-09)

## Description

### Introduction

In recent years, selective serotonin reuptake inhibitors (SSRIs) have started to be used for treating depression and other central nervous system disorders, noteworthy among which are fluoxetine, citalopram, sertraline and paroxetine. They all have different chemical structures, which helps to explain their different metabolic and phannacokinetic profiles. Their performance as antidepressants compares with that of classic tricyclic compounds, but their advantage is that they are safer and better tolerated.

The present invention relates to a number of new 4-substituted piperidines having an aryloxy functionality and potently inhibiting serotonin and/or noradrenaline reuptake, as a result of their high affinity for their neuronal transporters. This characteristic provides them with an enhanced antidepressant potential in human therapy. Other potential therapeutic applications of these compounds are treatment of nervous bulimia, alcohol addiction, anxiety, obsessive-compulsive disorders, panic, pain, pre-menstrual syndrome and social phobia, as well as migraine prophylaxis. Bibliography also describes other piperidine derivatives with aryloxy functionality as potential antidepressants, albeit with a chemical nature differing essentially from those claimed herein, since the piperidine is substituted at the 3-position. That is for instance the case of such compounds as 3-[(2-methoxyphenoxy)phenyl]methyl-piperidine 1 (Melloni, P., Carniel, G., Della Torre, A., Bonsignalari, A., Buonamici, M., Pozzi, O., Ricciardi, S., Rossi, A.C. Eur. J. Med Chem. Chim. Ther. 1984, 3, 235-242; Melloni, P., Della Torre, A., De Munari, S., Meroni, M., Tonani, R. Gazetta Chimica Italiana 1985, 115, 159-163) and 3-[(phenoxy)phenyl]methylpiperidine 2 (FR 2,010,615 CA73; 66442j; GB 1,203,149 CA73: 120509b). In these compounds, the substitution of the piperidine ring at the 3-position results in an additional chiral centre. The presence of the two chiral centres results in diastereomeric mixtures, which is the form in which the preparation of these compounds 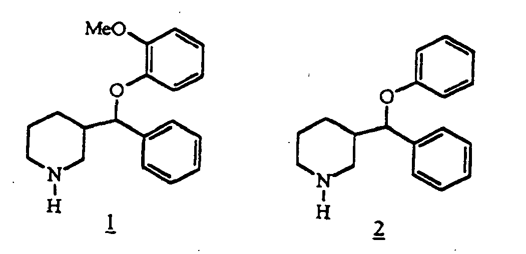 has been described. The preparation and/or isolation of pure enantiomers is not described in any case. However, the compounds claimed in the present specification possess a single chiral centre, since they haved the piperidine ring substituted at the 4-position. They have been prepared as racemic mixtures and as pure enantiomers, using synthetic methods differing from those in preparing 1 and 2.

Piperidine derivatives which are substituted at the ring nitrogen atom as well as in 4-position and which have serotonin antagonist activity are disclosed in Chemical Abstracts 106: 213767z and JP-A-61/227565.

Moreover, other piperidine derivatives having aryloxy functionality and the piperidine ring substituted at the 4-position have been described as potential antidepressants (formulae 3 and 4). Thus, in the case of 3 type compounds (JP 08/040999; CA 124: 343333n), 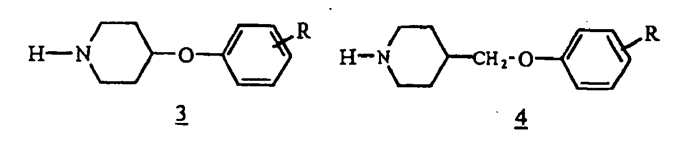 the aryloxy group is directly joined to the piperidine ring, whereas in the 4 type compounds (JP 08/040999; CA 124: 343333n) said group is joined to the piperidine ring through a methylene group which has no further substitutions. The compounds described herein differ largely from those, since they have the aryloxy group joined to the piperidine ring through a methylene group wherein, in all cases, one of the methylene group hydrogens is substituted by an aryl group, substituted or not, as defined hereinafter. These compounds are therefore structurally different from the 3 and 4 types and the synthetic methodology used in preparing the same is also absolutely different.

### Description

The new 4-substitude piperidines described in the present invention are represented by general formula (I), in which groups R₁ and R₂ are non-substituted aryl radicals or aryl radicals mono- or poly-substituted with halogen (fluorine, chlorine, bromine, iodine), alkyl, alkoxy, cyano, trifluoromethoxy, trifluoromethyl, benzoyl, phenyl, nitro, amino, aminoalkyl, aminoaryl and carbonylamino. 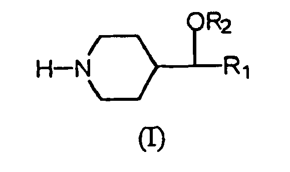 The compounds of general formula (I) have an asymmetric centre and have been prepared as racemic mixtures and as pure enantiomers. The present invention includes all optical isomers of the compounds of general formula (I) and racemic mixtures thereof.

The present invention further includes N,N-dimethyl-4-[[(4-piperidinyl)(phenyl)-methyl]oxy]-benzamide, obtained as a racemic mixture or as pure enantiomers. The present invention also comprises the pharmaceutically acceptable salts of the above-mentioned compounds with inorganic acids (such as: hydrochloric, hydrobromic, nitric, sulphuric and phosphoric) and with organic acids (such as: acetic, fumaric, tartaric, oxalic, citric, *p*-toluenesulphonic and methanosulphonic).

The racemic compounds of general formula (I) were prepared using well-known synthetic methods starting with the compounds of general formula (II).

Formation of the alkylarylether group was carried out using the Mitsunobu reaction (Mitsunobu, O. Synthesis 1981, 1; Hughes, D.L. Organic Reactions 42, 335) with phenols R₂-OH, in which R₂ is an aryl radical, substituted or not, as described for general formula (I), and the compounds of general formula (II), in which R₁ is an aryl radical, substituted or not, as described for general formula (I), and R₃ is hydrogen or R₄, which is an alkoxycarbonyl radical, preferably ethoxycarbonyl and t-butoxycarbonyl.

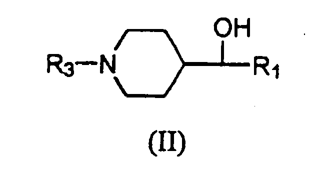

The alkylarylether group was also prepared using an aromatic nucleophilic substitution reaction (Berglund, R.A. Org. Proc. Res. Dev. 1997 1, 328-330) with the compounds of general formula (II) defined above, and the fluorinated derivatives R₂-F, in which R₂ is an aryl radical mono- or poly-substituted with halogen (fluorine, chlorine, bromine, iodine), alkyl, alkoxy, cyano, trifluoromethoxy, trifluoromethyl, benzoyl, phenyl, nitro, amino, aminoalkyl, aminoaryl and carbonylamino. The compounds of general formula (II) were prepared using conventional synthetic methods, starting with the compounds of general formula (III) (Duncan, R.L., Helsley, G.C., Welstead, WJ., DaVanzo, J.P., Funderburk, W.H., Lunsford, C.D. J. Med. Chem. 1970, 13 (1), 1), in which R₅ is an acetyl radical, ethoxycarbonyl and R₆ is cyano or carboxy.

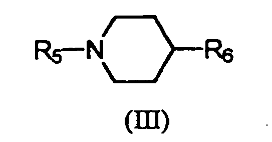

The compounds of general formula (III) defined above were transformed into the compounds of general formula (IV), in which R₁ is an aryl radical, substituted or not, 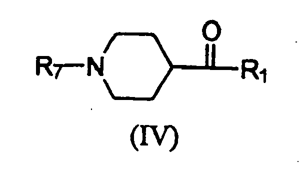 as described for the compounds of general formula (I), and R₇ is hydrogen, acetyl or R₄, which is an alkoxycarbonyl radical, preferably ethoxycarbonyl and t-butoxycarbonyl. Such transformation was made using two reaction types: a) a Friedel-Crafts reaction of the acid chlorides derived from the compounds of general formula (III), in which R₅ is an acetyl or ethoxycarbonyl and R₆ is carboxy (Duncan, R.L., Helsley, G.C., Welstead, W.J., DaVanzo, J.P., Funderburk, W.H., Lunsford, C.D. J. Med. Chem. 1970, 13 (1), 1) with benzene or conveniently functionalised derivatives thereof; or b) a Grignard reactive addition reaction, prepared from conveniently functionalised aryl halides, to compounds of general formula (III) in which R₅ is acetyl, ethoxycarbonyl or t-butoxycarbonyl and R₆ is cyano (Duncan, R.L., Helsley, G.C., Welstead, W.J., DaVanzo, J.P., Funderburk, W.H., Lunsford, C.D. J. Med. Chem. 1970, 13 (1), 1). Reduction of the compounds of general formula (IV) described provides the general formula (II) alcohols defined above.

The enantiomers composing the racemic mixtures of general formula (I) were obtained using two different pathways: a) resolution of the corresponding racemic mixture by split crystallisation of the diastereomeric salts prepared with chiral acids (D or L-dibenzoyltartaric, D or L-tartaric, D or L-di-p-toluyltartaric and D or L-mandelic) and b) enantioselective synthesis. In the latter case, the enantiomers of general formula (I) were obtained by reacting phenols R₂-OH or the fluorinated aromatic derivatives R₂-F defined above, with the enantiomers of the general formula (II) alcohols, as described for the racemic mixtures of general formula (I). In the enantiomers of the general formula (II) alcohols, R₁ is an aryl radical, substituted or not, as defined for the compounds of general formula (I), and R₃ is hydrogen or R₄, which is an alkoxycarbonyl radical, preferably ethoxycarbonyl and t-butoxycarbonyl. The enantiomers of the general formula (II) alcohols defined above were obtained by enantioselective reduction (Ramachandran, P.V., Teodorovic, A.V., Rangaishenvi, M.V., Brown, H.C. J. Org. Chem. 1992, 57, 2379-2386) of the compounds of general formula (IV) (Duncan, R.L., Helsley, G.C., Welstead, W.J., DaVanzo, J.P., Funderburk, W.H., Lunsford, C.D. J. Med. Chem. 1970, 13 (1), 1), in which R₁ is an aryl radical, substituted or not, as defined for the compounds of general formula (I), and R₇ is hydrogen or R₄, defined above.

The pharmacological activity of the compounds of general formula (I) was determined using well-established in vitro and in vivo pharmacological processes. The affinity of the compounds for the serotonin reuptake receptors (5HT) was evaluated in full rat cerebral cortex, using [³H]-paroxetine as radioligand (Habert, E., Graham, D., Tahraoui, L., Claustre, Y., Langer, S.Z. Eur J. Pharmacol. 1985, 118, 107-114) yielding Kᵢ values ranging between 0.5 and 500 nmol/l. The affinity of the compounds for noradrenaline (NA) reuptake receptors was evaluated in full rat cerebral cortex, using pH]-nisoxetine as radioligand (Tejani-Butt, S.M., J. Pharmacol. Exp. Ther. 1992, 260, 1, 427-436), yielding Kᵢ values ranging between 1 and 500 nmol/l. The following were used as assays predicting antidepressant activity: mouse tail suspension (Stéru, L., Chermat, R., Thierry, B., Mico, J.A., Lenégre, A., Stéru, M., Simon, P., Porsolt, R.D. Prog. Neurophsychopharmacol. Biol. Psychiat. 1987, 11, 659-671), rat or mouse desperate behaviour (Porsolt, R.D., Anton, G., Blavet, N., Jalfre, M. Eur. J Pharmacol. 1978, 48, 379-394) and enhancing rat yohimbine induced lethality (Quinton, R.M., Brit. J Pharmacol. 1963, 21, 51-66). The compounds with Kᵢ ranging between 0.5 and 40 nmol/l, for one of the transporters or for both, displayed an excellent antidepressant activity in the three models when administered within the 1 to 30 mg/Kg range orally, intraperitoneally or subcutaneously.

The following examples illustrate the scope of the present invention, which is not howsoever limited to such examples.

### Example 1

### (+/-)-4-[(4-trifluoromethoxyphenoxy)-2-(4-fluorophenyl) methyl]-piperidine, fumarate

A mixture of (+/-)-4-[(4-trifluoromethoxyphehyl)hydroxy]methyl-1-piperidinecarboxylic acid, 1,1-dimethyl-ethylester (2.25 g, 7.27 mmol), 2-pyridyl-diphenylphosphine (1.90 g, 7.27 mmol) and 1.3 g (7.4 mmol) of 4-trifluoromethoxyphenol in 40 mL of tetrahydrofurane (THF) was treated with a solution of diethyl-aza-dicarboxylate (DEAD) (1.15 mL) in 10 mL of THF. The reaction mixture was stirred at 20°C for 4-6 h and concentrated. The residue was dissolved in ethyl ether, washed with an aqueous HCl (10%) solution and an aqueous NaOH (5%) solution, dried (anh. Na₂SO₄), filtered and concentrated. 2.4 g (71%) were obtained of an oil which was dissolved in dichloromethane (50 mL) and treated with a solution of trifluoroacetic acid (2.1 mL) in 10 mL of dichloromethane. After h at 20°C, this was washed with an aqueous NaOH (5%) solution and saturated aqueous NaCl solution. Drying (anh. Na₂SO₄), filtering and concentration provided 1.3 g (71%) of the product, which was suspended in anhydrous ether (60 mL) and treated with fumaric acid (0.42 g), yielding 1.0 g of the fumarate (60% yield) with a m.p. = 130-134°C. The RMN-¹H (DMSO-d₆) displayed a characteristic signal at 4.31 ppm (d, *J* = 5.9 Hz, 1H, CHOAr) and RMN-¹³C (DMSO-d₆) displayed at 74.9 ppm a signal corresponding to CHOAr carbon.

### Example 2

### (+/-)-4-[(4-fluorophenoxy)(4-fluorophenyl) methyl]-piperidine, hydrochloride

A mixture of (+/-)-4-[(4-fluorophenyl)hydroxy]methyl-1-piperidinecarboxylic acid, 1,1-dimethyl-ethylester (16.33 mmol) and 1.9 g of 4-fluorophenol in 50 mL of THF was treated with 5.0 g of triphenylphosphine and a DEAD solution (3.45 mL) in 10 mL of THF was then added. After 3 h, the solvent was distilled and the resultant oil was treated with hexane, yielding a precipitate which was filtered. The filtrate was concentrated and the residue dissolved in dichloromethane (100 mL) and treated with a trifluoroacetic acid solution (8 mL) in 30 mL of dichloromethane. After 15 h, the reaction was worked as usual and the hydrochloride was prepared in THF, yielding 3.6 g thereof as an amorphous and slightly hygroscopic rose-coloured solid (Yield: 70%) with a m.p. = 90°C (d). RMN-¹H (CDCl₃) of the hydrochloride displayed a characteristic signal at 4.72 ppm (d,

*J* = 5.8 Hz, CHOAr) and RMN-¹³C (CDCl₃) a signal at 83.1 ppm corresponding to CHOAr carbon.

The following compounds were analogously prepared:
(+/-)-4-[(4-fluorophenoxy)(4-chlorophenyl) methyl]-piperidine, hydrochloride (54% yield, hygroscopic),
(+/-)-4-[(4-methoxyphenoxy)(4-fluorophenyl)methyl]-piperidine, fumarate (60% yield, m.p. = 139-142°C),
(+/-)-4-[(4-trifluoromethylphenoxy)(phenyl)methyl]-piperidine, hydrochloride (36% yield, hygroscopic),
(+/-)-4-[phenoxy)(4-chlorophenyl)(methyl]-piperidine, hydrochloride (72% yield, m.p. = 80°C (d)),
(+/-)-4-[(4-benzoylphenoxy)(phenyl)methyl]-piperidine, hydrochloride (74% yield, m.p. 70°C (d)),
and (+/-)-4-[(4-trifluoromethoxy)(phenyl)methyl]-piperidine, fumarate (58% yield, m.p. = 76°C (d)).

### Example 3

### (+/-)-4-[(4-fluorophenoxy)(phenyl)methyl]-piperidine, sulfate

An NaH (1.95 g, 60% mineral water) suspension in 20 mL of dimethylsulfoxide (DMSO) was treated with a solution of (+/-)-4-(phenylhydroxy)methyl-1-piperidinecarboxylic acid, 1,1-dimethyl-ethylester (13.8 g, 47 mmol) in 36 mL of DMSO. Potassium benzoate (7.5 g, 47 mmol) and 1.4-difluorobenzene (6.1 mL, 56 mmol) were added, and the reaction mixture was heated to 85°C until the starting substance disappeared. This was then treated with saturated aqueous NaCI and water solution, and extracted with ethyl ester. The organic phase evaporation residue was treated with methanol (200 mL) and aqueous HCl (10%, 200 mL) solution and refluxed for an hour. The product was isolated with the usual methodology, yielding an oil (9.6 g, 72% yield). RMN-¹H (CDCl₃) displayed a signal at 4.70 ppm (d, *J* = 7.1 Hz, CHOAr) and RMN-¹³C (CDCl₃) a signal at 85.0 ppm corresponding to CHOAr carbon. The oil was treated with a 1.85 mL conc. H₂SO₄ solution in 90 mL of water, yielding the sulfate as a solid with a m.p. = 118-120°C (75% yield).

### Example 4

### (+/-)4-[(3-fluorophenoxy)(phenyl)methyl]-piperidine, sulfate

An NaH (0.40 g, 60% mineral water) suspension in 6 mL DMSO was treated with a solution of (+/-)-4-(phenylhydroxy)methyl-1-piperidinecarboxylic acid, 1,1-dimethyl-ethylester (2.55 g, 8.75 mmol) in 6 mL of DMSO. Potassium benzoate (1.35 g, 8.43 mmol) and 1.3-difluorobenzene (1.05 mL, 10.6 mmol) were added, and the reaction mixture was heated to 85°C until the starting substance disappeared. It was then treated with saturated aqueous NaCl and water solution, and extracted with ethyl ester. The organic phase evaporation residue was treated with methanol (30 mL) and aqueous HCl (10%, 30 mL) solution and refluxed for an hour. The usual reaction working process yielded 2.16 g of an amber oil (88% yield). RMN-¹H (CDCl₃) displayed a signal at 4.78 ppm (d, *J* = 6.4 Hz, 1H, CHOAr) and RMN-¹³C (CDCl₃) a signal at 84.6 ppm corresponding to CHOAr carbon. The oil was treated with a 0.20 mL conc. H₂SO₄ solution in 10 mL of water, yielding the sulfate as a solid with a m.p. = 72-76°C.

The following compounds were analogously prepared:
(+/-)-4-[(phenoxy)(phenyl)methyl]piperidine, hydrochloride (73% yield, hygroscopic),
(+/-)-4-[(4-cyanophenoxy)(phenyl)methyl]-piperidine, fumarate (81% yield, m.p. = 76°C (d)),
(+/-)-4-[(3-trifluorophenoxy)(phenyl)methyl]-piperidine, hydrochloride (72% yield, m.p. = 58°C (d)),
(+/-)-4-[(4-bromophenoxy)(phenyl)methyl]-piperidine, sulfate (70% yield, m.p. = 99-103°C),
(+/-)-N,N-dimethyl-4-[[(4-piperidinyl)(phenyl)methyl]oxy]-benzamide, hydrochloride (72% yield, m.p. = 45°C (deliquescent)),
(+/-)-4-[(4-nitrophenyloxy)(phenyl)methyl]-piperidine, hydrochloride (80% yield, m.p. = 80°C (d)),
(+/-)-4-[(4-chlorophenyl)(1-naphthyloxy)methyl]-piperidine, sulfate (72% yield, m.p. = 186°C (d)),
(+/-)-4-[(1-naphthyloxy)(phenyl)methyl]-piperidine, sulfate (70% yield, m.p. = 152°C (d)),
(+/-)-4-[(2-fluorophenoxy)(phenyl)methyl]-piperidine, sulfate (72% yield, m.p. = 76°C (d)),
(+/-)-4-[(3-cyanophenoxy)(phenyl)methyl]-piperidine, hydrochloride (80% yield, m.p. = 82°C (d)),
(+/-)-4-[(3chlorophenoxy)(phenyl)methyl]-piperidine, sulfate (60% yield, m.p. = 101-104°C),
(+/-)-4-[(2-trifluoromethylphenoxy)(phenyl)methyl]-piperidine, sulfate (80% yield, m.p. = 110°C (d)),
(+/-)-4-[(2-cyanophenoxy)(phenyl)methyl]-piperidine, oxalate (80% yield, m.p. = 105°C (d)),
(+/-)-4-[[(2-biphenyl)oxy](phenyl)methyl]piperidine, hydrochloride (84% yield, m.p. = 84-87°C),
(+/-)-[[(4-biphenyl)oxy](phenyl)methyl]-piperidine, hydrochloride (82% yield, m.p. = 130°C (d)),
(+/-)-4-[(3-bromophenoxy)(phenyl)methyl]-piperidine, sulfate (75% yield, m.p. = 98°C (d)),
(+/-)-4-[(4-iodophenoxy)(phenyl)methyl]-piperidine, sulfate (57% yield, m.p. = 105°C (d)),
(+/-)4-[(3-iodophenoxy)(phenyl)methyl]piperidine, sulfate (37% yield, m.p. = 127°C (d)),
(+/-)-4-[(3,5-difluorophenoxy)(phenyl)methyl]piperidine, sulfate (86% yield, m.p. = 206-208°C),
(+/-)-4-[(3-fluoro-2-methylphenoxy)(phenyl)methyl]-piperidine, sulfate (80% yield, m.p. = 125°C (d)),
(+/-)-4-[(3-chloro-4-cyanophenoxy)(phenyl)methyl]-piperidine, hydrochloride (70% yield, m.p. = 125°C (d)),
(+/-)-4-[(5-chloro-2-methylphenoxy)(phenyl)methyl]-piperidine, sulfate (75% yield, m.p. = 105°C (d)),
(+/-)-4-[(3-chloro-2-methylphenoxy)(phenyl)methyl]-piperidine; sulfate (89% yield, m.p. = 130°C (d)),
(+/-)-4-[(3,4-dichlorophenoxy)(phenyl)methyl]piperidine, sulfate (91% yield, m.p. = 108°C (d)),
(+/-)4-[(3-methoxy-5-fluorophenoxy)(phenyl)methyl]-piperidine, hydrochloride (65% yield, m.p. = 200-203°C (d)), and
(+/-)-4-[(3-fluoro-5-cyanophenoxy)(phenyl)methyl]-piperidine, hydrochloride (76% yield, m.p. = 70°C (d)),

### Example 5

### Resolution of (+/-)-4-[(3-fluorophenoxy)(phenyl)methyl]-piperidine

4.45 g of L-(-)-dibenzoyltartaric acid were added over 7.1 g (25 mmol) of (+/-)-4-[3-fluorophenoxy)phenyl methyl-piperidine dissolved in 175 mL of ethanol (96%). A white solid was obtained (m.p. = 212°C (d)) which was treated with aqueous NaOH (5%) solution and extracted with chloroform, yielding the levorotary isomer (96% ee, m.p. = 59-62°C, [α]₅₄₆ - 11.4, c = 0.576, CHCl₃).

The filtrate liquids obtained were concentrated and the free base was extracted by treatment with aqueous NaOH (5%) solution and chloroform. The product obtained, dissolved in ethanol, was treated with D-(+)-dibenzoyltartaric acid using the preceding process. A white solid was obtained (m.p. = 208°C (d)) which was treated with aqueous NaOH (5%) solution and extracted with chloroform, yielding the dextrorotary isomer (98% ee, m.p. = 59-62°C, [α]₅₄₆ + 11.4, c = 0.618, CHCl₃).

The following compounds were analogously prepared:
(+)-4-[(4-fluorophenoxy)(phenyl)methyl]piperidine (96% ee, m.p. = 100-102°C, [α]₅₄₆ + 14, c = 0.259, CHCl₃)
(-)-4-[(4-fluorophenoxy)(phenyl)methyl]-piperidine (96% ee, m.p. = 100-102°C, [α]₅₄₆ - 14, c = 0.237, CHCl₃).
(+)-4-[(4-trifluoromethylphenoxy)(phenyl)methyl]-piperidine, sulfate (96% ee, m.p. = 85°C (d), [α]₃₆₅ + 17.8, c = 0.556, CHCl₃)
(-)-4-[(4-trifluoromethylphenoxy)(phenyl)methyl]-piperidine, sulfate (96% ee, m.p. = 85°C (d), [α] ₃₆₅ - 15.5, c = 0.508, CHCl₃)
(+)-4-[(4-bromophenoxy)(phenyl)methyl]-piperidine (96% ee, m.p. = 129-131°C (d), [α]₄₃₆ + 54, c =1.012,CHCl₃)
(-)-4-[(4-bromophenoxy)(phenyl)methyl]-piperidine (95% ee, m.p. = 129-131°C (d), [α]₄₃₆ - 54.1, c = 1.048, CHCl₃)
(+)-4-[(3-chlorophenoxy)(phenyl)methyl]-piperidine, methanosulfate (98% ee, m.p. = 200-202°C (d), [α]₃₆₅ + 14.6, c = 0.646, CHCl₃)
(-)-4-[(3-chlorophenoxy)(phenyl)methyl]-piperidine, methanosulfate (99% ee, m.p. = 200-202°C (d), [α]₃₆₅ + 13.6, c = 0.690, CHCl₃)
(+)-4-[(3-cyanophenoxy)(phenyl)methyl]-piperidine, hydrochloride (95% ee, m.p. = 70°C (d), [α]₄₃₆ + 26.5, c = 0.600, CHCl₃)
(-)-4-[(3-cyanophenoxy)(phenyl)methyl]piperidine, hydrochloride (98% ee, m.p. = 70°C (d), [α]₃₆₅ - 27.1, c = 0.680, CHCl₃)
(+)-4-[(3,5-difluorophenoxy)(phenyl)methyl]-piperidine, sulfate (96% ee, m.p. = 78°C (d), [α]₄₃₆ + 19.4, c = 0.80, CHCl₂)
(-)-4-[(3,5-difluorophenoxy)(phenyl)methyl]piperidine, sulfate (98% ee, m.p. = 78°C (d), [α]₄₃₆ - 19.8, c = 0.724, CHCl₃)
(+)-4-[(3-fluorophenoxy)(3-fluorophenyl) methyl]-piperidine, hydrochloride (96% ee, m.p. = 75°C (d), [α]₅₄₆ + 15, c = 0.183, CHCl₃) and
(-)-4-[(3-fluorophenoxy)(3-fluorophenyl) methyl]-piperidine, hydrochloride (95.4% ee, m.p. = 78°C (d), [α]₅₄₆ - 16, c.= 0.17, CHCl₃)

### Example 6

### (+)-4-[(4-fluorophenoxy)(phenyl)methyl]-piperidine

4-benzoyl-piperidine (2.0 g, 10.6 mmol) was added over a solution of 6.8 g of (+)-*B-*chlorodiisopinocanfeilboran ((+)-DIP-Cl) (21.25 mmol) in dichloromethane (20 mL, dry) cooled down to 3-4°C. After reacting for 72 h, 2.0 mL of acetaldehyde (35.46 mmol) were added and stirred at room temperature for 3 h. 24 mL of an aqueous NaOH (6N) solution, dichloromethane and saturated aqueous NaCI solution were added. The phases were separated and the usual treatment of the organic phase provided (+)-α-phenyl-4-piperidinemethanol as a white solid with a m.p. = 64-66°C in a 90% yield (84% ee).
1.8 g of aminoalcohol (+)-α-phenyl-4-piperidinemethanol (9.6 mmol) were dissolved in methanol (10 mL). The solution was cooled down to 0°C and a diterbutyl dicarbonate ((Boc)₂O) (2.5 g, 11.27 mmol) solution was added dropwise to 10 mL of methanol. The mixture was stirred for 24 h at room temperature, the methanol was concentrated, water was added and extracted with dichloromethane. The usual treatment of the organic phase provided the desired alcohol as a slightly coloured oil in a 93% yield.

The alcohol prepared above (2.7 g, 9.3 mmol) dissolved in DMSO (25 mL) was added over an NaH (60%, 0.6 g) suspension in DMSO (5 mL). Potassium benzoate (1.53 g, 9.63 mmol) and 1,4-difluorobenzene (1.3 mL, 11.9 mmol) were added and the mixture was heated (70-75°C) until the starting substance disappeared. The reaction mixture was poured into water and saturated aqueous NaCl solution, and extracted with ether. The oil obtained was refluxed with a mixture of methanol (40 mL) and an aqueous hydrochloric acid (40 mL) solution for 1h. Isolation of the product using the customary methodology provided (+)-4-[(4-fluorophenoxy)(phenyl)methyl]-piperidine as an oil in a 54% yield. Treatment of 0.5 g (1.75 mmol) of this oil with D-dibenzoyltartaric acid in ethanol (96%, 30 mL) provided a precipitate which was filtered (m.p. = 198-199°C). The aminoether was released yielding a white solid with a 96% ee, m.p. = 102-104°C, and [α]₅₄₆ + 15, c = 0.105, CHCl₃).

The following compounds were analogously prepared:
(+)4-[(4-nitrophenoxy)(phenyl)methyl]-piperidine, hydrochloride (96% ee, m.p. = 55°C (d), [α]₄₃₆ + 36, c = 0.045, Ethanol)
(-)-4-[(1-naphthyloxy)(phenyl)methyl]-piperidine, hydrochloride (98% ee, m.p. = 65°C (d), [α]₅₄₆ - 180, c = 0.080, CHCl₃) and
(+)-4-[(2-fluorophenoxy)(phenyl)methyl]-piperidine, sulfate (97.6% ee, m.p. = 105°C (d), [α]₅₄₆ + 31, c = 0.081, CHCl₃).

### Example 7

### (-)-4-[(4-fluorophenoxy)(phenyl)methyl]-piperidine

4-benzoyl-piperidine (7.35 g, 39.05 mmol) was added over a solution of 25 g of (-)-DIP-Cl (78.125 mmol) in dichloromethane (75 mL, dry) cooled down to 0-2°C. After reacting for 72 h, 5.2 mL of acetaldehyde (92.2 mmol) were added and stirred at room temperature for 3 h. 71 mL of an aqueous NaOH (6N) solution, dichloromethane and saturated aqueous NaCl solution were added. The phases were separated and the usual treatment of the organic phase provided (-)-α-phenyl-4-piperidinemethanol as a white solid with a m.p. = 48-50°C in a 85% yield (86% ee). 2 g of aminoalcohol (-)-α-phenyl-4-piperidinemethanol (10.7 mmol) were dissolved in methanol (10 mL). The solution was cooled down to 0°C and a (Boc)₂O (2.6 g, 11.73 mmol) solution was added dropwise to 7 mL of methanol. The mixture was stirred for 20 h at room temperature, the methanol was concentrated, water was added and extracted with dichloromethane. The usual treatment of the organic phase provided the desired alcohol as a slightly coloured oil in a 90% yield.

The alcohol prepared above (1.3 g, 4.5 mmol) dissolved in DMSO (10 mL) was added over an NaH (60%, 210 g) suspension in DMSO (5 mL). Potassium benzoate (715 g, 4.5 mmol) and 1,4-difluorobenzene (0.75 mL, 6.86 mmol) were added and the mixture heated (70-75°C) until the starting substance disappeared. The reaction mixture was poured into water and saturated aqueous NaCI solution, and extracted with ether. The oil obtained was refluxed with a mixture of methanol (17 mL) and an aqueous hydrochloric acid (17 mL) solution for 1h. The usual working of the reaction provided (-)-4-[(4-fluorophenoxy)(phenyl)methyl]-piperidine as an oil in a 64% yield. Treatment of this oil with L-dibenzoyltartaric acid in ethanol (96%, 35 mL) provided a precipitate which was filtered (m.p. = 193-194°C). The aminoether was released yielding a white solid with a 98% ee, m.p. = 100-102°C, and [α]₅₄₆ - 14, c = 0.2, CHCl₃).

The following compounds were analogously prepared:
(-)-4-[(4-nitrophenoxy)(phenyl)methyl]-piperidine, hydrochloride (98.7% ee, m.p. = 59°C (d), [α]₄₃₆ - 31, c = 0.042, Ethanol)
(+)-4-[(1-naphthyloxy)(phenyl)methyl]-piperidine, hydrochloride (94% ee, m.p. = 115°C (d), [α]₅₄₆ - + 156, c = 0.128, CHCl₃) and
(-)4-[(2-fluorophenoxy)(phenyl)methyl]-piperidine, sulfate (97.6% ee, m.p. = 90°C (d), [α]₅₄₆ - 31, c = 0.140, CHCl₃).

### Example 8

### (+/-)-[(3-fluorophenoxy)(3-fluorophenyl) methyl]-piperidine, sulfate

a mixture of 4-cyanopiperidine (5 g, 40.92 mmol), (Boc)₂O (11.7 g, 53.7 mmol), sodium bicarbonate (11.7 g, 139.3 mmol) and water (117 mL) was stirred at room temperature for 17 h. this was extracted with dichloromethane and the organic phase dried (anh. Na₂SO₄), filtered and concentrated. The resultant oil was purified by flash chromatography (Still, W.C., Kahn, M., Mitra, A. J. Org. Chem. 1978, 43, 2923) yielding 4-cyano-1-piperidinecarboxylic acid, 1,1-dimethyl-ethylester as a yellow oil in a 43% yield.

A Mg (0.5 g) suspension in ether (dry, 22mL) was treated with some millilitres (approximately ¼ of the total) of a 1-bromo-3-fluorobenzene (2.15 mL, 19.4 mmol) solution in ether (dry, 16 mL) and an iodine crystal. This was heated until a smooth reflux was observed and the colour disappeared. The rest of the solution was then added dropwise maintaining a mild reflux. With the addition at an end, this was refluxed for 1 h 30 min and allowed to cool down to room temperature. A 4-cyano-1-piperidinecarboxylic acid ,1,1-dimethyl-ethylester (2.7 g, 12.84 mmol) solution was added dropwise to dry ether (27 mL) and the resultant mixture refluxed for 3 h. A saturated aqueous NH₄Cl (50 mL) solution was added and extracted with ether. The usual treatment of the organic phase provided an oil which was purified by flash chromatography (Still, W.C., Kahn, M., Mitra, A. J. Org. Chem. 1978, 43, 2923) yielding 2.4 g (61% yield) of 4-(3-fluorobenzoyl)-1-piperidinecarboxylic acid, 1,1-dimethyl-ethylester as a yellowish oil. The product obtained above (2.4 g, 7.8 mmol) was dissolved in methanol (30 mL) and NaBH₄ (0.2 g) dissolved in 3.5 mL water was added. The mixture was heated for 2 h in an oil bath (50-60°C) and the product isolated in the usual manner, yielding (+/-)4-(3-fluorophenyl)hydroxy]methyl-1-piperidinecarboxylic acid, 1,1-dimethyl-ethylester as a very dense yellowish oil in quantitative yield.

A solution of the racemic alcohol prepared above (2.4 g, 7.8 mmol) in DMSO (25 mL) was added dropwise to an NaH (60%) (0.62 g) suspension in DMSO (15 mL). Potassium benzoate (1.53 g, 9.55 mmol) and 1,3-difluorobenzene (1.2 mL, 11.9 mmol) were added and the mixture was heated in an oil bath (65-70°C) until the starting substance disappeared. This was then poured into a mixture of saturated NaCl (50 mL) solution and water (39 mL). This was extracted with ether and the usual treatment of the ethereal phase provided an oil which was refluxed with a mixture of methanol (40 mL) and aqueous HCI (10 %, 40 mL) solution for 1 h 30 min. The desired product (+/-)4-[(3-fluorophenoxy)(3-fluorophenyl)methyl]-piperidine was obtained as an amber oil in a 50% yield. RMN-¹H (CDCl₃) of this product displayed a signal at 4.55 ppm (d, *J* = 6.1 Hz, CHOAr) and RMN-¹³C (CDCl₃) a signal at 83.9 ppm corresponding to CHOAr carbon. The oil prepared above was treated with a 0.22 mL conc. H₂SO₄ solution in 16.5 mL of water, yielding the sulfate as a slightly coloured solid (m.p. = 158°C (d)).

The following compounds were analogously prepared:
(+/-)-4-[(2-fluorophenoxy)(3-fluorophenyl) methyl]-piperidine, hydrochloride (62% yield, m.p. = 90°C) and
(+/-)-4-[(4-fluorophenoxy)(3-fluorophenyl) methyl]-piperidine, hydrochloride (30% yield, m.p. = 65°C).

## Claims

1. 4-substituted piperidines, of general formula (I), in which R₁ and R₂ are non-substituted aryl radicals or aryl radicals mono- or polysubstituted with halogen (fluorine, chlorine, bromine, iodide), alkyl, alkoxy, cyano, trifluromethoxy, trifluoromethyl, benzoyl, phenyl, nitro, amino, aminoalkyl, aminoaryl and carbonylamino,
and their pharmaceutically acceptable salts with inorganic acids and organic acids.

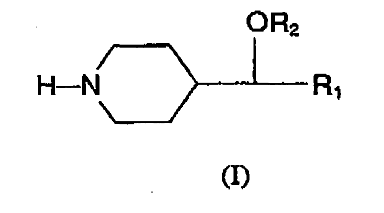

2. 4-substituted piperidines according to claim 1, **characterized in that** the 4-substituted piperidines of general formula (I)are obtained as racemic mixtures or as pure enantiomers.

3. 4-substituted piperidines according to claim 1 or 2, **characterized in that** they are 4-substituted piperidines of general formula (I) as listed herein after, obtained as racemic mixtures or pure enantiomers, and their pharmaceutically acceptable salts:
4-[(phenoxy)(phenyl)methyl]-piperidine,
4-[(4-fluorophenoxy)(phenyl)methyl]-piperidine,
4-[(4-methoxyphenoxy)(4-fluorophenyl)methyl]-piperidine,
4-[(4-fluorophenoxy)(4-fluorophenyl)methyl]-piperidine,
4-[(4-fluorophenoxy)(4-chlorophenyl)methyl]-piperidine,
4-[(4-trifluoromethylphenoxy)(phenyl)methyl]-piperidine,
4-[(4-trifluoromethoxyphenoxy)(4-fluorophenyl)methyl]-piperidine,
4-[(phenoxy)(4-chlorophenyl)methyl]-piperidine,
4-[(4-benzoylphenoxy)(phenyl)methyl]-piperidine,
4-[(4-trifluoromethoxyphenoxy)(phenyl)methyl]-piperidine,
4-[(4-cyanophenoxy)(phenyl)methyl]-piperidine,
4-[(3-trifluorophonoxy)(phenyl)methyl]-piperidine,
4-[(3-fluorophenoxy)(phenyl)methyl]-piperidine,
4-[(4-bromophenoxy)(phenyl)methyl]-piperidine,
4-[(4-nitrophenyloxy)(phenyl)methyl]-piperidine,
4-[(4-chlorophenyl)(1-naphthyloxy)methyl]-piperidine,
4-[(1-naphthyloxy)(phenyl)methyl]-piperidine,
4-[(2-fluorophenoxy)(phenyl)methyl]-piperidine,
4-[(3-cyanophenoxy)(phenyl)methyl]-piperidine,
4-[(3-chlorophenoxy)(phenyl)methyl]-piperidine,
4-[(2-trifluoromethylphenoxylphenoxyl)methyl]-piperidine,
4-[(2-cyanophenoxy)(phenyl)methyl]-piperidine,
4-[[(2-biphenyl)oxy](phenyl)methyl]-piperidine,
4-[(3-fluorophenoxy)(3-fluorophenyl)methyl]-piperidine,
4-[(2-fluorophenoxy)(3-fluorophenyl)methyl]-piperidine,
4-[(4-fluorophenoxy)(3-fluorophenyl)methyl]-piperidine,
4-[[(4-biphenyl)oxy](phenyl)methyl]-piperidine,
4-[(3-bromophenoxy)(phenyl)methyl]-piperidine,
4-[(4-iodophenoxy)(phenyl)methyl]-piperidine,
4-[(3-iodophenoxy)(phenyl)methyl]-piperidine,
4-[(3,5-difluorophenoxy)(phenyl)methyl]-piperidine,
4-[(3-fluoro-2-methylphenoxy)(phenyl)methyl]-piperidine,
4-[(3-chloro-4-cyanophenoxy)(phenyl)methyl]-piperidine,
4-[(5-chloro-2-methylphenoxy)(phenyl)methyl]-piperidine,
4-[(3-chloro-2-methylphenoxy)(phenyl)methyl]-piperidine,
4-[(3,4-dichlorophenoxy)(phenyl)methyl]-piperidine,
4-[(3-methoxy-5-fluorophenoxy)(phenyl)methyl]-piperidine, and
4-[(3-fluoro-5-cyanophenoxy)(phenyl)methyl]-piperidine.

4. 4-[(3-fluorophenoxy)(phenyl)methyl]-piperidine according to claim 3 obtained as a racemic mixture or as pure enantiomers, and the pharmaceutically acceptable salts of said compound.

5. N,N-dimethyl-4-[[(4-piperidinyl)(phenyl)methyl]oxy]-benzamide, obtained as a racemic mixture or as pure enantiomers, and the pharmaceutically acceptable salts of said compounds with inorganic acids and with organic acids.

6. Pharmaceutical compositions containing a therapeutically effective quantity of a compound of general formula (I) in accordance with claims 1 to 3, or of the compounds claimed in claim 4 or in claim 5, mixed with pharmaceutically acceptable excipients, to be orally, parenterally and topically administered.

7. Use of a compound of general formula (I) in accordance with claims 1 to 3, or of the compounds claimed in claim 4 or in claim 5, for the manufacture of a medicament for treating central nervous system disorders in humans, particularly depression, nervous bulimia, obsessive-compulsive disorders, alcohol addiction, anxiety, panic, pain, pre-menstrual syndrome, social phobia, and for migraine prophylaxis.

## Patentansprüche

1. 4-Substituierte Piperidine der allgemeinen Formel (I), wobei R₁ und R₂ nicht-substituierte Arylreste oder Arylreste, welche mono- oder polysubstituiert mit Halogen (Fluor, Chlor, Brom, Iod), Alkyl, Alkoxy, Cyano, Trifluormethoxy, Trifluormethyl, Benzoyl, Phenyl, Nitro, Amino, Aminoalkyl, Aminoaryl und Carbonylamino sind, darstellen,
und ihre pharmazeutisch verträglichen Salze mit anorganischen Säuren und organischen Säuren.

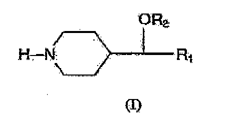

2. 4-Substituierte Piperidine gemäß Anspruch 1, **dadurch gekennzeichnet, dass** die 4-substituierten Piperidine der allgemeinen Formel (I) als racemische Gemische oder als reine Enantiomere erhalten werden.

3. 4-Substituierte Piperidine gemäß Anspruch 1 oder 2, **dadurch gekennzeichnet, dass** sie 4-substituierte Piperidine der allgemeinen Formel (I) wie nachstehend angegeben, erhalten als racemische Gemische oder reine Enantiomere, und ihre pharmazeutisch verträglichen Salze, sind:
4-[(Phenoxy)(phenyl)methyl]piperidin,
4-[(4-Fluorphenoxy)(phenyl)methyl]piperidin,
4-[(4-Methoxyphenoxy)(4-fluorphenyl)methyl]piperidin,
4-[(4-Fluorphenoxy)(4-fluorphenyl)methyl]piperidin,
4-[(4-Fluorphenoxy)(4-chlorphenyl)methyl]piperidin,
4-[(4-Trifluormethylphenoxy)(phenyl)methyl]piperidin,
4-[(4-Tritluormethoxyphenoxy)(4-fluorphenyl)methyl]piperidin,
4-[(Phenoxy)(4-chlorphenyl)methyl]piperidin,
4-[(4-Benzoylphenoxy)(phenyl)methyl]piperidin,
4-[(4-Trifluormethoxyphenoxy)(phenyl)methyl]piperidin,
4-[(4-Cyanophenoxy)(phenyl)methyl]piperidin,
4-[(3-Trifluorphenoxy)(phenyl)methyl]piperidin,
4-[(3-Fluorphenoxy)(phenyl)methyl]piperidin,
4-[(4-Bromphenoxy)(phenyl)methyl]piperidin,
4-[(4-Nitrophenyloxy)(phenyl)methyl]piperidin,
4-[(4-Chlorphenyl)(1-naphthyloxy)methyl]piperidin,
4-[(1-Naphthyloxy)(phenyl)methyl]piperidin,
4-[(2-Fluorphenoxy)(phenyl)methyl]piperidin,
4-[(3-Cyanophenoxy)(phenyl)methyl]piperidin,
4-[(3-Chlorphenoxy)(phenyl)methyl]piperidin,
4-[(2-Trifluormethylphenoxy)(phenyl)methyl]piperidin,
4-[(2-Cyanophenoxy)(phenyl)methyl]piperidin,
4-[[(2-Biphenyl)oxy](phenyl)methyl]piperidin,
4-[(3-Fluorphenoxy)(3-fluorphenyl)methyl]piperidin,
4-[(2-Fluorphenoxy)(3-fluorphenyl)methyl]piperidin,
4-[(4-Fluorphenoxy)(3-fluorphenyl)methyl]piperidin,
4-[[(4-Biphenyl)oxy](phenyl)methyl]piperidin,
4-[(3-Bromphenoxy)(phenyl)methyl]piperidin,
4-[(4-Iodphenoxy)(phenyl)methyl]piperidin,
4-[(3-Iodphetioxy)(phenyl)methyl]piperidin,
4-[(3,5-Difluorphenoxy)(phenyl)methyl]piperidin,
4-[(3-Fluor-2-methylphenoxy)(phenyl)methyl]piperidin,
4-[(3-Chlor-4-cyanophenoxy)(phenyl)methyl]piperidin,
4-[(5-Chlor-2-methylphenoxy)(phenyl)methyl]piperidin,
4-[(3-Chlor-2-methylphenoxy)(phenyl)methyl]piperidin,
4-[(3,4-Dichlorphenoxy)(phenyl)methyl]piperidin,
4-[(3-Methoxy-5-fluorphenoxy)(phenyl)methyl]piperidin und
4-[(3-Fluor-5-cyanophenoxy)(phenyl)methyl]piperidin.

4. 4-[(3-Fluorphenoxy)(phenyl)methyl]piperidin gemäß Anspruch 3, erhalten als ein racemisches Gemisch oder als reine Enantiomere, und die pharmazeutisch verträglichen Salze dieser Verbindung.

5. N,N-Dimethyl-4-[[(4-piperidinyl)(phenyl)methyl]oxy]benzamid, erhalten als ein racemisches Gemisch oder als reine Enantiomere, und die pharmazeutisch verträglichen Salze dieser Verbindung mit anorganischen Säuren und mit organischen Säuren.

6. Arzneimittel, enthaltend eine therapeutisch wirksame Menge einer Verbindung der allgemeinen Formel (I) gemäß den Ansprüchen 1 bis 3 oder der Verbindung gemäß Anspruch 4 oder Anspruch 5, gemischt mit pharmazeutisch verträglichen Exzipienten, zur oralen, parenteralen und topischen Verabreichung.

7. Verwendung einer Verbindung der allgemeinen Formel (I) gemäß den Ansprüchen 1 bis 3 oder der Verbindung gemäß Anspruch 4 oder Anspruch 5 zur Herstellung eines Medikaments zur Behandlung von Erkrankungen des zentralen Nervensystems in Menschen, insbesondere Depression, nervöser Bulimie, Zwangsneurosen, Alkoholabhängigkeit, Angst, Panik, Schmerz, prämenstruellem Syndrom, Sozialphobie, und zur Migräneprophylaxe.

## Revendications

1. Pipéridines substituées en position 4, de formule générale (I), où R₁ et R₂ sont des radicaux aryles non substitués ou des radicaux aryles mono- ou poly-substitués par un halogène (fluor, chlore, brome, iode), un alkyle, un alcoxy, un cyano, un trifluorométhoxy, un trifluorométhyle, un benzoyle, un phényle, un nitro, un amino, un aminoalkyle, un aminoaryle et un carbonylamino, et leurs sels pharmaceutiquement acceptables avec des acides inorganiques et organiques.

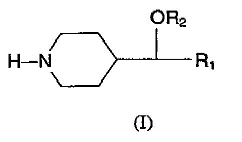

2. Pipéridines substituées en position 4 selon la revendication 1, **caractérisées en ce que** les pipéridines substituées en position 4 de formule générale (I) sont obtenues sous forme de mélanges racémiques ou d'énantiomères purs.

3. Pipéridines substituées en position 4 selon la revendication 1 ou 2, **caractérisées en ce qu'**il s'agit de pipéridines substituées en position 4 de formule générale (I) telles qu'énumérées dans la liste suivante, obtenues sous forme de mélanges racémiques ou d'énantiomères purs, et leurs sels pharmaceutiquement acceptables :
4-[(phénoxy)(phényl)méthyl]-pipéridine,
4-[(4-fluorophénoxy)(phényl)méthyl]-pipéridine,
4-[(4-méthoxyphénoxy)(4-fluorophényl)méthyl]-pipéridine,
4-[(4-fluorophénoxy)(4-fluorophényl)méthyl]-pipéridine,
4-[(4-fluorophénoxy)(4-chlorophényl)méthyl]-pipéridine,
4-[(4-trifluorométhylphénoxy)(phényl)méthyl]-pipéridine,
4-[(4-trifluorométhoxyphénoxy)(4-fluorophényl)méthyl]-pipéridine,
4-[(phénoxy)(4-chlorophényl)méthyl]-pipéridine,
4-[(4-benzoylphénoxy)(phényl)méthyl]-pipéridine,
4-[(4-trifluorométhoxyphénoxy)(phényl)méthyl]-pipéridine,
4-[(4-cyanophénoxy)(phényl)méthyl]-pipéridine,
4-[(3-trifluorophénoxy)(phényl)méthyl]-pipéridine,
4-[(3-fluorophénoxy)(phényl)méthyl]-pipéridine,
4-[(4-bromophénoxy)(phényl)méthyl]-pipéridine,
4-[(4-nitrophényloxy)(phényl)méthyl]-pipéridine,
4-[(4-chlorophényl)(1-naphthyloxy)méthyl]-pipéridine,
4-[(1-naphthyloxy)(phényl)méthyl]-pipéridine,
4-[(2-fluorophénoxy)(phényl)méthyl]-pipéridine,
4-[(3-cyanophénoxy)(phényl)méthyl]-pipéridine,
4-[(3-chlorophénoxy)(phényl)méthyl]-pipéridine,
4-[(2-trifluorométhylphénoxy)(phényl)méthyl]-pipéridine,
4-[(2-cyanophénoxy)(phényl)méthyl]-pipéridine,
4-[[(2-biphényl)oxy](phényl)méthyl]-pipéridine,
4-[(3-fluorophénoxy)(3-fluorophényl)méthyl]-pipéridine,
4-[(2-fluorophénoxy)(3-fluorophényl)méthyl]-pipéridine,
4-[(4-fluorophenoxy)(3-fluorophényl)méthyl]-pipéridine,
4-[[(4-biphényl)oxy](phényl)méthyl]-pipéridine,
4-[(3-bromophénoxy)(phényl)méthyl]-pipéridine,
4-[(4-iodophénoxy)(phényl)méthyl]-pipéridine.
4-[(3-iodophénoxy)(phényl)méthyl]-pipéridine,
4-[(3,5-difluorophénoxy)(phényl)méthyl]-pipéridine,
4-[(3-fluoro-2-méthylphénoxy)(phényl)méthyl]-pipéridine,
4-[(3-chloro-4-cyanophénoxy)(phényl)méthyl]-pipéridine,
4-[(5-chloro-2-méthylphénoxy)(phényl)méthyl]-pipéridine,
4-[(3-chloro-2-méthylphénoxy)(phényl)méthyl]-pipéridine,
4-[(3,4-dichlorophénoxy)(phényl)méthyl]-pipéridine,
4-[(3-méthoxy-5-fluorophénoxy)(phényl)méthyl]-pipéridine, et
4-[(3-fluoro-5-cyanophénoxy)(phényl)méthyl]-pipéridine.

4. 4-[(3-fluorophénoxy)(phényl)méthyl]-pipéridine selon la revendication 3 obtenu sous forme de mélange racémique ou d'énantiomères purs, et les sels pharmaceutiquement acceptables dudit composé.

5. N,N-diméthyl-4-[[(4-pipéridinyl)(phényl)méthyl]oxy]-benzamide, obtenu sous forme de mélange racémique ou d'énantiomères purs, et les sels pharmaceutiquement acceptables dudit composé avec des sels inorganiques et organiques.

6. Compositions pharmaceutiques contenant une quantité thérapeutiquement efficace d'un composé de formule générale (I) selon les revendications 1 à 3, ou du composé revendiqué dans la revendication 4 ou dans la revendication 5, mélangé avec des excipients pharmaceutiquement acceptables, pour être administré par voie orale, parentérale et topique.

7. Utilisation d'un composé de formule générale (I) selon les revendications 1 à 3, ou du composé revendiqué dans la revendication 4 ou dans la revendication 5, pour la fabrication d'un médicament destiné à traiter les troubles du système nerveux central chez les êtres humains, en particulier la dépression, la boulimie nerveuse, les troubles obsessionnels-compulsifs, l'alcoolisme, l'anxiété, la panique, la douleur, le syndrome prémenstruel, la phobie sociale et la prophylaxie de la migraine.
